# EUROPÄISCHE PATENTSCHRIFT

(11) **EP 0 806 243 B1**
(45) Veröffentlichungstag und Bekanntmachung des Hinweises auf die Patenterteilung: **30.08.2000**
(21) Anmeldenummer: 97107011.5
(22) Anmeldetag: 28.04.1997
(51) Int. Cl.: B01J 23/96, C07C 68/00, B01J 38/48, B01J 38/60

(54) **Verfahren zur Reaktivierung von Platinmetall enthaltenden Katalysatorsystemen**
Method for reactivation of a platinum group metal catalytic system
Procédé pour la réactivation d'un système catalytique contenant un métal du groupe du platine

(30) Priorität: 10.05.1996 DE 19618829
(43) Veröffentlichungstag der Anmeldung: 12.11.1997
(73) Patentinhaber: BAYER AG, 51368 Leverkusen (DE)
(72) Erfinder: Buysch, Hans-Josef, Dr., 47809 Krefeld (DE); Hesse, Carsten, Dr., 47800 Krefeld (DE); Rechner, Johann, Dr., 47906 Kempen (DE)

(56) Entgegenhaltungen:
- US-A- 3 488 295

## Beschreibung

Die vorliegende Erfindung betrifft ein Verfahren zur Reaktivierung von Platinmetall enthaltenden Katalysatorsystemen die aus mindestens einem Platinmetall, einem Cokatalysator und weiteren Salzen bestehen, die zur Herstellung von Diarylcarbonaten durch oxidative Umsetzung von aromatischen Hydroxyverbindungen mit Kohlenmonoxid eingesetzt werden, das dadurch gekennzeichnet ist, daß man das desaktivierte Katalysatorsystem mit einem Oxidationsmittel behandelt, das überschüsssige Oxidationsmittel abtrennt und das reaktivierte Katalysatorsystem mit einem Diketonat versetzt.

Es ist bekannt, Diarylcarbonate durch oxidative Umsetzung von aromatischen Hydroxyverbindungen mit Kohlenmonoxid in Gegenwart eines Edelmetall-Katalysators herzustellen (DE-OS 28 15 512). Als Edelmetall wird bevorzugt Palladium eingesetzt. Zusätzlich können ein Cokatalysator (z.B. Mangan- oder Kobaltsalze), eine Base, ein quartäres Salz, verschiedene Chinone bzw. Hydrochinone und Trockenmittel eingesetzt werden. Dabei kann in einem Lösungsmittel, bevorzugt Methylenchlorid, gearbeitet werden.

Bei kontinuierlicher Prozeßführung ist bei Verfahren dieser Art ein Absinken der Raum-Zeit-Ausbeute zu beobachten, das auf eine Desaktivierung des homogenen Katalysatorsystems zurückzuführen ist. Desaktivierte Katalysatorbestandteile fallen dabei als Pulver aus dem Reaktionssystem aus. Diese Pulver zeigen nur noch sehr geringe oder gar keine katalytische Aktivität mehr. Die Literatur enthält keinerlei Angaben zur Reaktivierung dieses Katalysatorsystems. Zum Erhalt einer hohen Raum-Zeit-Ausbeute müssen desaktivierte Katalysatoranteile aus dem Prozeß ausgeschleust und durch frischen Katalysator ersetzt werden. Weil der Edelmetall-Katalysator einen erheblichen Kostenfaktor darstellt und Verluste an Edelmetall-Katalysator entsprechend kostenintensiv ersetzt werden müssen, hängt die Wirtschaftlichkeit eines Diarylcarbonat-Herstellungsverfahrens mit homogenem Katalysatorsystem stark vom Verbrauch an Platinmetall/Cokatalysator ab. Durch Reaktivierung desaktivierter Katalysatorsysteme könnte der Frischkatalysator-Bedarf des Prozesses drastisch gesenkt werden, so daß eine wirtschaftliche Durchführung möglich ist. Es bestand daher die Aufgabe, ein einfaches Verfahren zu finden, mit dem desaktivierte Katalysatorsysteme reaktiviert werden können.

Es wurde nun gefunden, daß desaktivierte, Platinmetall enthaltende Katalysatorsysteme, bestehend aus mindestens einem Platinmetall, einem Cokatalysator und weiteren Salzen, die zur Herstellung von Diarylcarbonaten durch oxidative Umsetzung von aromatischen Hydroxyverbindungen mit Kohlenmonoxid eingesetzt werden, durch Behandlung mit einem Oxidationsmittel und anschließendem Zusatz eines Diketonats reaktiviert werden können. Das nicht verbrauchte, überschüssige Oxidationsmittel kann zurückgewonnen und wieder eingesetzt werden.

Die Erfindung betrifft demnach ein Verfahren zur Reaktivierung von desaktivierten Katalysatoren zur oxidativen Carbonylierung von aromatischen Hydroxyverbindungen, die ein Platinmetall und ein cokatalytisch wirkendes Metall enthalten, das dadurch gekennzeichnet ist, daß man die desaktivieren Katalysatoren bei 10-400°C in flüssiger Phase mit 1-10 000 Äquivalenten von Oxidationsmitteln pro Äquivalent der metallischen Bestandteile des Katalysatorsystems behandelt, überschüssiges Oxidationsmittel abtrennt und den dabei zurückbleibenden oxidierten Rückstand mit 0,1-100 Gew.-Teilen eines C₁-C₁₂-Carboxylats oder eines C₄-C₁₂-Diketonats, bezogen auf 1 Gew.-Teil des oxidierten Rückstands umsetzt.

Es wird angenommen, daß sich hierbei in Phenol lösliche Carboxylate bzw. Diketonate bilden.

Katalytisch wirkende Metalle und ihre Verbindungen sind solche der Platinmetallgruppe, wie Ru, Rh, Pd, Ir oder Pt, bevorzugt Pd.

Cokatalytisch wirkende Metalle und ihre Verbindungen sind solche der Gruppen III B, IV B, V B, VI B, VII B, VIII B, I B, II B (CAS-Nomenklatur) oder ein Gemisch mehrerer von ihnen z.B. Mangan, Kupfer, Kobalt, Vanadium, bevorzugt Mn.

Die erfindungsgemäße Behandlung der desaktivierten Katalysatorbestandteile mit einem Oxidationsmittel erfolgt bei 10 bis 350°C, bevorzugt bei 20 bis 250°C, besonders bevorzugt bei 30 bis 200°C.

Bei den in das erfindungsgemäße Verfahren einsetzbaren Oxidationsmittel handelt es sich um solche Verbindungen oder Elemente, die unter den Reaktionsbedingungen Elektronen aus den desaktivierten Katalysatorbestandteilen aufnehmen; so nimmt beispielsweise bei Säuren das Proton H⁺ ein Elektron auf gemäß 2H⁺ + 2 e^{⊖} → H₂.

Beispiele für solche Oxidationsmittel sind solche aus der Gruppe der starken Mineralsäuren, der elementaren Halogene sind O₂, O₃, Peroxi- und Hydroperoxiverbindungen, Nitrate, Permanganate, Halogenate, Perhologenate.

Das Verhältnis von Oxidationsmittel zu Katalysatorrückstand beträgt im erfindungsgemäßen Verfahren für flüssige Oxidationsmittel 10000:1, bevorzugt 1000:1, besonders bevorzugt 500:1. Gasförmige Oxidationsmittel werden mit einer Rate von 0,01 bis 5000, bevorzugt 0,1 bis 500, besonders bevorzugt 1 bis 100 Normalliter pro Gramm Katalysatorrückstand und Stunde dosiert.

Für diesen Schritt des erfindungsgemäßen Verfahrens wird eine Zeit von 0,5 bis 20 h, bevorzugt 0,5 bis 10 h, besonders bevorzugt 1 bis 8 h benötigt.

Feste Oxidationsmittel (z.B. Jod, KMnO₄, KClO₄ u.a.) werden in einem der unten genannten Lösungsmittel gelöst.

Flüssige Oxidationsmittel (z.B. Brom, H₂SO₄ u.a.) können pur oder verdünnt mit einem polaren Lösungsmittel (s.u.) eingesetzt werden.

Gasförmige Oxidationsmittel (z.B. Cl₂, O₂, O₃, Halogenwasserstoffe u.a.) werden als Lösung in einem der unten genannten Lösungsmittel eingesetzt oder durch eine Suspension des desaktivierten Katalysators in einem der unten genannten Lösungsmittel geperlt.

In einer möglichen Ausführungsform des erfindungsgemäßen Verfahrens wird der Katalysatorrückstand zur Behandlung mit gasförmigen Oxidationsmittel (z.B. Luft oder Chlor) in einem der oben beschriebenen Lösungsmittel aufgeschlämmt und das gasförmige Oxidationsmittel durch die Lösung geleitet. Dies kann bei einem Druck von 0,8 bis 100 bar bevorzugt 0,9 bis 50 bar, besonders bevorzugt 0,9 bis 10 bar erfolgen. Nach der Umsetzung wird das Lösungsmittel bevorzugt durch Destillation in bekannter Weise, wobei Temperatur und Druck in weiten Grenzen variiert werden können, ohne daß der Katalysator geschädigt wird.

Werden flüssige Oxidationsmittel verwendet, kann die Behandlung des Katalysatorrückstands mit Oxidationsmittel unter Inertgasatmosphäre (Stickstoff, Argon, o.ä.), an der Luft oder unter der für die oxidative Carbonylierung organischer Hydroxyverbindungen üblichen Atmosphäre (Kohlenmonoxid/Luft, Kohlenmonoxid/Sauerstoff) drucklos oder unter Druck durchgeführt werden. Überschüssiges Oxidationsmittel kann beispielsweise destillativ, auch unter vermindertem Druck oder durch Zerstörung mit einem Reduktionsmittel entfernt werden.

Bevorzugt sind Oxidationsmittel aus der Gruppe der starken Mineralsäuren, besonders bevorzugt aus der Gruppe von H₂SO₄, HNO₃ und Halogenwasserstoffen, ganz besonders bevorzugt aus der Gruppe der Halogenwasserstoffe. Insbesondere werden die Halogenwasserstoffe als wäßrige Halogenwasserstoffsäuren mit einer Konzentration von 5 bis 70 Gew.-%, bevorzugt 10 bis 60 Gew.-%, besonders bevorzugt 15 bis 50 Gew.-% Halogenwasserstoff am Gesamtgewicht der Säure, bevorzugt als wäßrige Bromwasserstoffsäure eingesetzt.

Zur Ausbildung der flüssigen Phase werden polare Lösungsmittel verwendet.

Als polare Lösungsmittel sind solche geeignet, die mit dem vorgesehenen Oxidationsmittel nicht reagieren. Beispiele für solche Lösungsmittel sind solche aus der Gruppe von Wasser, C₁-C₄-Carbonsäuren, C₁-C₄-Carbonsäureestern mit einer C₁-C₄-Estergruppe, C₁-C₄-Carbonsäureamiden mit einer -NH₂-, -NH(C₁-C₄-alkyl)- oder -N(C₁-C₄-alkyl)₂-Gruppe, aliphatischen C₁-C₆-Mono-, Di- oder Polyolen, (cyclo)aliphatischen C₁-C₆-Mono- oder Diketonen und (cyclo)aliphatischen oder aromatischen C₂-C₇-Nitrilen, die in einer Menge von 1 bis 1 000 Gewichtsteilen, bezogen auf 1 Gewichtsteil eingesetztes desaktiviertes Katalysatorpulver, bevorzugt 2 bis 500 besonders bevorzugt 5 bis 250 Gewichtsteile eingesetzt werden. Bevorzugt wird als polares Lösungsmittel ein wasserhaltiges Gemisch mit einem H₂O-Anteil von 10-99 % am Gesamtgewicht des Gemisches, beispielsweise mit Essigsäure oder Propionsäure oder Wasser, bevorzugt Wasser eingesetzt.

Im letzten Schritt des erfindungsgemäßen Verfahrens wird der zurückbleibende, oxidierte Rückstand aus der Abtrennung des überschüssigen Oxidationsmittels und des polaren Lösungsmittels mit einem Carboxylat von C₁-C₁₂-Carbonsäuren oder einem C₄-C₁₂-Diketonat, bevorzugt mit einem Acetat oder Acetylacetonat umgesetzt Geeignete Carboxylate sind z.B. Formiate, Acetate, Propionate, Butyrate, Pentanoate, Hexanoate oder Capronate, mit Kationen aus der Gruppe von Li, Na, K, Mg, Ca, Mn, Fe, Co, Ce und Platingruppenmetall, das im zu reaktivierenden Katalysator enthalten ist, wie Natriumformiat, NaOAc, (mit OAc = Acetat) KOAc, Natriumpropionat, Natriumbutyrat, Natriumpentanoat, Natriumhexanoat oder Natriumcapronat, Erdalkalimetallcarboxylate wie Mg(OAc)₂ oder Ca(OAc)₂ oder Übergangsmetallcarboxylate wie Mn(OAc)₃, Mn(OAc)₂, Fe(OAc)₂, Co(OAc)₂, Ce(OAc)₃ oder Pd(OAc)₂. Geeignete Diketonate sind solche mit den genannten Kationen und sind beispielsweise Alkalimetallacetylacetonate wie Li(acac) mit acac = Acetylacetonat, Na(acac), K(acac), Rb(acac) und Cs(acac), Erdalkalimetallacetylacetonate wie Mg(acac)₂ oder Ca(acac)₂ oder Übergangsmetallacetylacetonate wie Cr(acac)₃, Mn(acac)₂, Mn(acac)₃, Fe(acac)₂, Fe(acac)₃, Co(acac)₂, Co(acac)₃ Ce(acac)₃ oder Pd(acac)₂.

Die Menge des zugesetzten Carboxylats oder Diketonats beträgt im erfindungsgemäßen Verfahren 0,1 bis 100, bevorzugt 0,2 bis 50, besonders bevorzugt 0,5 bis 25 Gew.-Teile pro Gew.-Teil oxidierter Katalysatorrückstand nach Abtrennung des Oxidationsmittels.

Der durch das erfindungsgemäße Verfahren reaktivierte Katalysator hat eine Aktivität, die größer als 95% des Frischkatalysators ist.

Die folgenden Beispiele verdeutlichen das erfindungsgemäße Verfahren, ohne es jedoch auf diese einzuschränken.

### Beispiel 1:

### Reaktivierung:

2 g eines desaktivierten Katalysator-Pulvers (Gehalt nach Atomabsorptionsspektrometrie: 33,9% Palladium, 14,5% Mangan, Rest zu 100 %:Na⁺- und Br⁻-Ionen), das aus einem vorangegangenen Semibatch-Ansatz zur Herstellung von Diphenylcarbonat (DPC; 10 bar Reaktionsdruck, 65 ppm, Pd, etwa 500 ppm Mn) stammte, wurden mit 50 ml einer 48 Gew.-%igen wäßrigen Bromwasserstofflösung versetzt und auf 100°C erhitzt. Nach einer Stunde lag eine homogene Lösung vor. Anschließend wurde bei einem Druck von 30 mbar und einer Temperatur von 80°C die HBr-Lösung am Rotavapor abdestilliert. Die Rückgewinnungsrate der HBr betrug 98%. Der Rückstand wurde an der Luft getrocknet und wog 3,22 g (Gewichtszunahme durch Br-Aufnahme). Die mit Hilfe eines Rotationsverdampfers zurückgewonnene HBr konnte direkt wiederverwendet werden.

### Wiedereinsatz in die Katalyse:

In einem Autoklaven (1 l) mit Begasungsrührer, Kühler und nachgeschalteter Kühlfalle wurden 0,65 g des reaktivierten Katalysators (=136 mg Palladium und 58 mg Mangan) und 8,31 g Tetrabutylammoniumbromid bei 80°C in 450 g Phenol gelöst. Dann wurden 0,51 g Mangan(II)acetylacetonat und 2,21 g Natriumphenolat, gelöst in 50 g Phenol, zugegeben und unter Einleitung eines Gasgemisches aus Kohlenmonoxid und Sauerstoff (96,5:3,5 Vol.%) der Druck auf 10 bar eingestellt. Der Mangangehalt in der Reaktionslösung betrug 330 ppm. Die Menge an Gasgemisch wurde auf 260 Nl/h eingestellt. Dem Reaktionsgemisch wurde jede Stunde eine Probe entnommen und gaschromatographisch analysiert.

Die Analysen ergaben, daß nach einer Stunde 7,4 % Diphenylcarbonat, nach 2 Stunden 12,2 % Diphenylcarbonat und nach 3 Stunden 17,4 % Diphenylcarbonat im Reaktionsgemisch enthalten waren; das entspricht einer Aktivität von durchschnittlich 98,0 % des erstmals eingesetzten Frischkatalysators.

### Vergleichsversuch mit Frischkatalysator:

In einem Autoklaven (1 l) mit Begasungsrührer, Kühler und nachgeschalteter Kühlfalle wurden 0,34 g Palladiumbromid (=136 mg Palladium) und 8,31 g Tetrabutylammoniumbromid bei 80°C in 450 g Phenol gelöst. Durch diese Lösung wurde zur Aktivierung des Katalysators eine Stunde lang Kohlenmonoxid (3 l/h) geleitet. Dann wurden 0,77 g Mangan(II)acetylacetonat und 2,21 g Natriumphenolat, gelöst in 50 g Phenol, zugegeben und unter Einleitung eines Gasgemisches aus Kohlenmonoxid und Sauerstoff (96,5:3,5 Vol.%) der Druck auf 10 bar eingestellt. Der Mangangehalt in der Reaktionslösung betrug 330 ppm. Die Menge an Gasgemisch wurde auf 260 Nl/h eingestellt. Dem Reaktionsgemisch wurde jede Stunde eine Probe entnommen und gaschromatographisch analysiert.

Die Analysen ergaben, daß nach einer Stunde 7,6% Diphenylcarbonat, nach 2 Stunden 12,4% Diphenylcarbonat und nach 3 Stunden 17,7% Diphenylcarbonat im Reaktionsgemisch enthalten waren.

### Beispiel 2:

Es wurde wie in Beispiel 1 verfahren, jedoch wurde das desaktivierte Katalysatorpulver nicht mit 48 Gew.-%iger wäßriger Bromwasserstofflösung, sondern mit 98%iger Schwefelsäure behandelt. Nach einer Stunde bei 100°C lag eine homogene Lösung vor. Die Schwefelsäure wurde durch Vakuumdestillation zu 95% zurückgewonnen. Der resultierende reaktivierte Katalysator zeigte beim Wiedereinsatz in den Prozeß der oxidativen Carbonylierung aromatischer Hydroxyverbindungen eine Aktivität von 96% des Frischkatalysators.

### Beispiel 3:

Es wurde wie in Beispiel 1 verfahren, jedoch wurde das desaktivierte Katalysatorpulver nicht mit 48 Gew.-%iger wäßriger Bromwasserstofflösung, sondern mit 65 %iger Salpetersäure behandelt. Nach einer Stunde bei 80°C lag eine homogene Lösung vor. Die Salpetersäure wurde durch Vakuumdestillation zu 99 % zurückgewonnen. Der resultierende reaktivierte Katalysator zeigte beim Wiedereinsatz in den Prozeß der oxidativen Carbonylierung aromatischer Hydroxyverbindungen eine Aktivität von 98% des Frischkatalysators.

### Beispiel 4:

Es wurde wie in Beispiel 1 verfahren, jedoch wurde das desaktivierte Katalysatorpulver nicht mit 48 Gew.-%iger wäßriger Bromwasserstofflösung, sondern mit 24 Gew.-%iger wäßriger Bromwasserstofflösung behandelt. Nach einer Stunde bei 100°C lag eine homogene Lösung vor. Die Bromwasserstoffsäure wurde durch Vakuumdestillation zu 98 % zurückgewonnen. Der resultierende reaktivierte Katalysator zeigte beim Wiedereinsatz in den Prozeß der oxidativen Carbonylierung aromatischer Hydroxyverbindungen eine Aktivität von 98% des Frischkatalysators.

### Beispiel 5:

Es wurde wie in Beispiel 1 verfahren, jedoch wurde dem resultierenden reaktivierten Katalysator nach der Rückgewinnung der Bromwasserstoffsäure 1,9 g (= 15,5 mmol) Natriumacetylacetonat zugesetzt.

### Wiedereinsatz in die Katalyse:

In einem Autoklaven (1 l) mit Begasungsrührer, Kühler und nachgeschalteter Kühlfalle wurden 1,03 g des reaktivierten Katalysators (=136 mg Palladium und 58 mg Mangan) und 8,31 g Tetrabutylammoniumbromid bei 80°C in 450 g Phenol gelöst. Dann wurden 0,51 g Mangan(II)acetylacetonat und 2,21 g Natriumphenolat, gelöst in 50 g Phenol, zugegeben und unter Einleitung eines Gasgemisches aus Kohlenmonoxid und Sauerstoff (96,5:3,5 Vol.%) der Druck auf 10 bar eingestellt. Der Mangangehalt in der Reaktionslösung betrug 330 ppm. Die Menge an Gasgemisch wurde auf 260 Nl/h eingestellt. Dem Reaktionsgemisch wurde jede Stunde eine Probe entnommen und gaschromatographisch analysiert.

Die Analysen ergaben, daß nach einer Stunde 7,5 % Diphenylcarbonat, nach 2 Stunden 12,3 % Diphenylcarbonat und nach 3 Stunden 17,5 % Diphenylcarbonat im Reaktionsgemisch enthalten waren; das entspricht einer Aktivität von durchschnittlich 98,9 % des Frischkatalysators.

## Patentansprüche

1. Verfahren zur Reaktivierung von desaktivierten Katalysatoren zur oxidativen Carbonylierung von aromatischen Hydroxyverbindungen, die ein Platinmetall und ein cokatalytisch wirkendes Metall enthalten, dadurch gekennzeichnet, daß man die desaktivieren Katalysatoren bei 10-400°C in flüssiger Phase mit 1-10 000 Äquivalenten von Oxidationsmitteln pro Äquivalent der metallischen Bestandteile des Katalysatorsystems behandelt, überschüssiges Oxidationsmittel abtrennt und den dabei zurückbleibenden oxidierten Rückstand mit 0,1-100 Gew.-Teilen eines C₁-C₁₂-Carboxylats oder eines C₄-C₁₂-Diketonats, bezogen auf 1 Gew.-Teil des oxidierten Rückstands versetzt.

2. Verfahren nach Anspruch 1, dadurch gekennzeichnet, daß das Platinmetall Palladium ist.

3. Verfahren nach Anspruch 1, dadurch gekennzeichnet, daß ein cokatalytisch wirkendes Metall in gebundener Form aus der Gruppe III B, IV B, V B, VI B, VII B, VIII B, I B, II B oder ein Gemisch mehrerer von ihnen, bevorzugt Mn, vorliegt.

4. Verfahren nach Anspruch 1, dadurch gekennzeichnet, daß ein Oxidationsmittel aus der Gruppe der starken Mineralsäuren, der elementaren Halogene, O₂, O₃, Peroxi- und Hydroperoxiverbindungen, Nitraten, Permanganaten, Halogenaten, Perhalogenaten, bevorzugt aus der Gruppe der starken Mineralsäuren, besonders bevorzugt aus der Gruppe von H₂SO₄, HNO₃ und Halogenwasserstoffen, ganz besonders bevorzugt aus der Gruppe der Halogenwasserstoffe, in einer Menge von 1-10 000 Äquivalenten Oxidationsmitteln pro Äquivalent der metallischen Bestandteile des zu reaktivierenden Katalysators eingesetzt wird.

5. Verfahren nach Anspruch 4, dadurch gekennzeichnet, daß die Halogenwasserstoffe als wäßrige Halogenwasserstoffsäuren mit einer Konzentration von 5-70 Gew.-%, bevorzugt 10-60 Gew.-%, besonders bevorzugt 15-50 Gew.-% Halogenwasserstoff am Gesamtgewicht der Säure, bevorzugt als wäßrige Bromwasserstoffsäure eingesetzt werden (wird).

6. Verfahren nach Anspruch 1, dadurch gekennzeichnet, daß als polares Lösungsmittel eines oder mehrere aus der Gruppe von Wasser, C₁-C₄-Carbonsäuren, C₁-C₄-Carbonsäureestern mit einer C₁-C₄-Estergruppe, C₁-C₄-Carbonsäureamiden mit einer -NH₂-, -NH(C₁-C₄-alkyl)- oder -N(C₁-C₄-alkyl)₂-Gruppe, aliphatischen C₁-C₆-Mono-, Di- oder Polyolen, (cyclo)aliphatischen C₁-C₆-Mono- oder Diketonen und (cyclo)aliphatischen oder aromatischen C₂-C₇-Nitrilen in einer Menge von 1 bis 1 000 Gewichtsteilen, bevorzugt 2 - 500, besonders bevorzugt 5 - 250 Gewichtsteilen, bezogen auf 1 Gewichtsteil desaktivierten Katalysator, eingesetzt wird.

7. Verfahren nach Anspruch 6, dadurch gekennzeichnet, daß als polares Lösungsmittel ein wasserhaltiges Gemisch mit einem H₂O-Anteil von 10-99 % am Gesamtgewicht des Gemisches oder Wasser, bevorzugt Wasser eingesetzt wird.

8. Verfahren nach Anspruch 1, dadurch gekennzeichnet, daß als Carboxylate und Diketonate Salze mit Kationen aus der Gruppe von Alkalimetallen, Erdalkalimetallen, Übergangsmetallen und dem Platingruppenmetall, das im zu reaktivierenden Katalysator enthalten ist, bevorzugt aus der Gruppe von Li, Na, K, Mg, Ca, Mn, Fe, Co, Ce und dem Platingruppenmetall, das im zu reaktivierenden Katalysator enthalten ist, eingesetzt werden.

9. Verfahren nach Anspruch 1, dadurch gekenzeichnet, daß man 1 bis 1 000, bevorzugt 1 bis 500 Äquivalente Oxidationsmittel pro Äquivalent der metallischen Bestandteile des zu reaktivierenden Katalysators einsetzt.

10. Verfahren nach Anspruch 1, dadurch gekennzeichnet, daß man 0,2 bis 50, bevorzugt 0,5 bis 25 Gew.-Teile Carboxylat oder Diketonat, bezogen auf 1 Gew.-Teil des oxidierten Rückstandes, einsetzt.

## Claims

1. A process for reactivating deactivated catalysts for the oxidative carbonylation of aromatic hydroxy compounds which contain a platinum group metal and a co-catalytically active metal, characterised in that the deactivated catalyst is treated at 10 - 400°C in the liquid phase with 1 - 10000 equivalents of oxidising agent per equivalent of metallic consituents in the catalyst system, excess oxidising agent is separated therefrom and 0.1 - 100 parts by weight of a C₁-C₁₂ carboxylate or a C₄-C₁₂ diketonate, with respect to 1 part by weight of the oxidised residue, is added to the remaining oxidised residue.

2. A process according to Claim 1, characterised in that the platinum group metal is palladium.

3. A process according to Claim 1, characterised in that a co-catalytically active metal from group III B, IV B, V B, VI B, VII B, VIII B, I B, II B or a mixture of several of these, preferably Mn, is present in bonded form.

4. A process according to Claim 1, characterised in that an oxidising agent from the group of strong inorganic acids, elemental halogens, O₂, O₃, peroxy and hydroperoxy compounds, nitrates, permanganates, halogenates, perhalogenates, preferably from the group of strong mineral acids, more preferably from the group consisting of H₂SO₄, HNO₃ and hydrogen halides, most preferably from the group of hydrogen halides, is used in an amount of 1 - 10000 equivalents of oxidising agent per equivalent of metallic constituents in the catalyst being reactivated.

5. A process according to Claim 4, characterised in that the hydrogen halide(s) is (are) used as aqueous hydrohalic acids with a concentration of 5 - 70 wt.%, preferably 10 - 60 wt.%, in particular 15 - 50 wt.% of hydrogen halide to total weight of acid, preferably as aqueous hydrobromic acid.

6. A process according to Claim 1, characterised in that one or more of the group consisting of water, C₁-C₄ carboxylic acids, C₁-C₄ carboxylates with a C₁-C₄ ester group, C₁-C₄ carboxylic amides with a -NH₂, -NH(C₁-C₄ alkyl), or -N(C₁-C₄ alkyl)₂ group, aliphatic C₁-C₆ mono, di or polyols, (cyclo)aliphatic C₁-C₆ mono or diketones and (cyclo)aliphatic or aromatic C₂-C₇ nitriles in an amount of 1 to 1000 parts by weight, preferably 2 - 500, in particular 5 - 250 parts by weight, with respect to 1 part by weight of deactivated catalyst, is used as a polar solvent.

7. A process according to Claim 6, characterised in that a water-containing mixture with a proportion of H₂O of 10 - 99 % in the total weight of the mixture or water, preferably water, is used as a polar solvent.

8. A process according to Claim 1, characterised in that salts with cations from the group consisting of alkali metals, alkaline earth metals, transition metals and the platinum group metal which is contained in the catalyst being reactivated, preferably from the group consisting of Li, Na, K, Mg, Ca, Mn, Fe, Co, Ce and the platinum group metal which is contained in the catalyst being reactivated, are used as carboxylates and diketonates.

9. A process according to Claim 1, characterised in that 1 to 1000, preferably 1 to 500 equivalents of oxidising agent are used per equivalent of metallic constituents in the catalyst being reactivated.

10. A process according to Claim 1, characterised in that 0.2 to 50, preferably 0.5 to 25 parts by weight of carboxylate or diketonate, with respect to 1 part by weight of oxidised residue, are used.

## Revendications

1. Procédé de réactivation de catalyseurs désactivés pour la carbonylation oxydative de composés hydroxylés aromatiques, qui contiennent un métal du groupe du platine et un métal agissant comme cocatalyseur, caractérisé en ce que le catalyseur désactivé est traité à 10-400°C en phase liquide, avec 1-10 000 équivalents d'agent d'oxydation par équivalent de constituant métallique du système catalytique, on sépare l'agent d'oxydation en excès et on fait réagir le résidu oxydé subsistant avec 0,1-100 parties en poids d'un carboxylate en C₁-C₁₂ ou un dicétonate en C₄-C₁₂, sur base de 1 partie en poids du résidu oxydé.

2. Procédé suivant la revendication 1, caractérisé en ce que le métal du groupe du platine est le palladium.

3. Procédé suivant la revendication 1, caractérisé en ce qu'un métal agissant comme cocatalyseur est présent sous forme liée et appartient au groupe IIIB, IVB, VB, VIB, VIIB, VIIIB, IB, IIB ou un mélange de plusieurs de ceux-ci, de préférence Mn.

4. Procédé suivant la revendication 1, caractérisé en ce que l'agent d'oxydation choisi parmi le groupe des acides minéraux forts, des halogènes élémentaires, de O₂, de O₃, des composés peroxydes et hydroperoxydes, des nitrates, des permanganates, des halogénates, des perhalogénates, de préférence le groupe des acides minéraux forts, de manière particulièrement préférée le groupe de H₂SO₄, HNO₃ et des halogénures d'hydrogène, de manière tout particulièrement préférée le groupe des halogénures d'hydrogène, est mis en oeuvre en une quantité allant de 1 à 10 000 équivalents d'agent d'oxydation par équivalent de constituant métallique du catalyseur à réactiver.

5. Procédé suivant la revendication 4, caractérisé en ce que l'halogénure d'hydrogène est mis en oeuvre sous forme d'acide halogénohydrique aqueux avec une concentration allant de 5 à 70% en poids, de préférence de 10 à 60% en poids, de manière particulièrement préférée de 15 à 50% en poids d'halogénure d'hydrogène par rapport au poids total de l'acide, de préférence de l'acide bromhydrique aqueux.

6. Procédé suivant la revendication 1, caractérisé en ce que l'on met en oeuvre comme solvant polaire, un ou plusieurs choisis parmi le groupe de l'eau, des acides carboxyliques en C₁-C₄, des esters d'acide carboxylique en C₁-C₄ avec un radical ester en C₁-C₄, des amides d'acide carboxylique en C₁-C₄ avec un radical -NH₂-, -NH(alcoyl en C₁-C₄)- ou -N(alcoyl en C₁-C₄)₂-, les mono-, di- ou polyols aliphatiques en C₁-C₆, les mono- ou dicétones (cyclo)aliphatiques en C₁-C₆, et les nitriles (cyclo)aliphatiques ou aromatiques en C₂-C₇, en une quantité allant de 1 à 1000 parties en poids, de préférence de 2 à 500, de manière particulièrement préférée de 5 à 250 parties en poids, sur base de une partie en poids du catalyseur désactivé.

7. Procédé suivant la revendication 6, caractérisé en ce que l'on met en oeuvre comme solvant polaire, un mélange aqueux avec une quantité de H₂O allant de 10 à 99% par rapport au poids total du mélange, ou de l'eau, de préférence de l'eau.

8. Procédé suivant la revendication 1, caractérisé en ce que l'on met en oeuvre comme carboxylate et dicétonate, des sels avec des cations choisis parmi le groupe des métaux alcalins, des métaux alcalino-terreux, des métaux de transition et le métal du groupe du platine, qui est contenu dans la catalyseur à réactiver, de préférence parmi le groupe Li, Na, K, Mg, Ca, Mn, Fe, Co, Ce et un métal du groupe du platine qui est contenu dans le catalyseur à réactiver.

9. Procédé suivant la revendication 1, caractérisé en ce que l'on met en oeuvre 1 à 1000, de préférence 1 à 500 équivalents d'agent d'oxydation par équivalent de constituant métallique du catalyseur à réactiver.

10. Procédé suivant la revendication 1, caractérisé en ce que l'on met en oeuvre 0,2 à 50, de préférence 0,5 à 25 parties en poids de carboxylate ou dicétonate, sur base de 1 partie en poids du résidu oxydé.
